(19) Europäisches Patentamt
European Patent Office
Office européen des brevets

(11) **EP 1 165 764 B1**

(12) **EUROPÄISCHE PATENTSCHRIFT**

(45) Veröffentlichungstag und Bekanntmachung des Hinweises auf die Patenterteilung:
**01.05.2013 Patentblatt 2013/18**

(51) Int Cl.:
*C12N 11/02* (2006.01)   *C07K 14/215* (2006.01)
*C07K 17/02* (2006.01)

(21) Anmeldenummer: **00929335.8**

(22) Anmeldetag: **31.03.2000**

(86) Internationale Anmeldenummer:
**PCT/EP2000/002904**

(87) Internationale Veröffentlichungsnummer:
**WO 2000/058450 (05.10.2000 Gazette 2000/40)**

(54) **LINKERFREIE KOVALENTE KOPPLUNG VON BAKTERIORHODOPSIN IN PURPURMEMBRANFORM**

LINKER-FREE COVALENT COUPLING OF BACTERIORHODOPSIN IN PURPLE MEMBRANE FORM

COUPLAGE COVALENT SANS LIEUR DE BACTERIORHODOPSINE SOUS FORME DE MEMBRANE POURPRE

(84) Benannte Vertragsstaaten:
**AT BE CH CY DE DK ES FI FR GB GR IE IT LI LU MC NL PT SE**

(30) Priorität: **31.03.1999 DE 19914702**
**08.11.1999 DE 19953607**

(43) Veröffentlichungstag der Anmeldung:
**02.01.2002 Patentblatt 2002/01**

(73) Patentinhaber: **U-NICA Technology AG**
**7208 Malans (CH)**

(72) Erfinder:
• **HAMPP, Norbert**
**35287 Amöneburg (DE)**
• **SEITZ, Arne**
**D-35037 Marburg (DE)**
• **PASTERNACK, Ralf**
**D-64347 Griesheim (DE)**
• **FUCHSBAUER, H.-L.**
**D-64367 Mühltal/Traisa (DE)**

(74) Vertreter: **Detken, Andreas et al**
**Isler & Pedrazzini AG**
**Gotthardstrasse 53**
**Postfach 1772**
**8027 Zürich (CH)**

(56) Entgegenhaltungen:
EP-A- 0 417 541    EP-A- 0 532 029
EP-A- 0 726 317    WO-A-99/06446
US-A- 5 922 843

• **B.J. GAFFNEY: "Chemical and biochemical crosslinking of membrane components" BIOCHIMICA BIOPHYSICA ACTA, Bd. 822, 1985, Seiten 289-317, XP000863159 ELSEVIER SCIENCE, AMSTERDAM, NL**
• **SINGH A K ET AL: "Photoactive bacteriorhodopsin variants" RADIATION PHYSICS AND CHEMISTRY,NL,ELSEVIER SCIENCE PUBLISHERS BV., AMSTERDAM, Bd. 49, Nr. 1, 1997, Seiten 131-134, XP004014865 ISSN: 0969-806X**
• **BRAUCHLE C ET AL: "OPTICAL APPLICATIONS OF BACTERIORHODOPSIN AND ITS MUTATED VARIANTS" ADVANCED MATERIALS,DE,VCH VERLAGSGESELLSCHAFT, WEINHEIM, Bd. 3, Nr. 9, 1. September 1991 (1991-09-01), Seiten 420-428, XP000329344 ISSN: 0935-9648**
• **BRAEUCHLE C ET AL: "OPTICAL INFORMATION PROCESSING WITH BACTERIORHODOPSIN AND ITS GENETICALLY MODIFIED VARIANTS" PROCEEDINGS OF THE QUANTUM ELECTRONICS AND LASER SCIENCE CONFERENCE,US,NEW YORK, IEEE, Bd. CONF. 4, 2. Mai 1993 (1993-05-02), Seite 2 XP000376687 ISBN: 1-55752-301-0**
• **MIGNEAULT ISABELLE ET AL: "Glutaraldehyde: behavior in aqueous solution, reaction with proteins, and application to enzyme crosslinking", BIOTECHNIQUES, INFORMA HEALTHCARE, US, vol. 37, no. 5, 1 November 2004 (2004-11-01), pages 790-796,798, XP001539341, ISSN: 0736-6205**

**Beschreibung**

Die Erfindung betrifft die linkerfreie kovalente Kopplung von Bakteriorhodopsin (BR) in membrangebundener Form, insbesondere in Purpurmembran-Form.

Bakteriorhodopsin (BR) ist ein Membranprotein, das z.B. in Halobakterien in der Form eines zweidimensionalen, scheibenförmigen, wasserunlöslichen Kristalls, der sogenannten Purpurmembran (PM) vorkommt. Die Purpurmembran hat eine Dicke von ca. 5 nm. Der Durchmesser der Purpurmembran liegt typischerweise im Bereich von 300 nm bis 1000 nm.

Die Purpurmembran besteht aus $\alpha$-helikalen Bakteriorhodopsinmolekülen und ca. 10 Lipidmolekülen pro Bakteriorhodopsinmolekül. Das Bakteriorhodopsin ist in die aus den Lipidmolekülen gebildete Lipidmembran fast vollständig eingebettet. Daraus ergibt sich seine ungewöhnliche thermodynamische Stabilität.

Bakteriorhodopsin in Purpurmembran-Form wird als photochromes Material für optische Anwendungen technisch genutzt. Weiter ist Bakteriorhodopsin in Purpurmembran-Form als Material mit photoelektrischen Eigenschaften von technischer Bedeutung.

Für diese technischen Anwendungen ist die kovalente Quervernetzung von Purpurmembranen untereinander vorteilhaft. Bei der Verwendung von Bakteriorhodopsin in der Photovoltaik werden pro Purpurmembranschicht etwa 300 mV erhalten. Durch die Bildung von mehreren Purpurmembran-schichten übereinander mittels kovalenter Quervernetzung können Elemente erhalten werden, die höhere Spannungen liefern. Ein weiterer interessanter Aspekt ist die kovalente Kopplung von Purpurmembranen an Oberflächen, Polymere oder kleinere Moleküle, z.B. Farbstoffe.

Üblicherweise versucht man, für die kovalente Bindung von Purpurmembranen niedermolekulare Linkermoleküle, z.B. Glutaraldehyd, einzusetzen. Allerdings verlaufen diese Kopplungsreaktionen mit sehr schlechter Ausbeute. Der Grund dafür ist, dass nur wenige Aminosäuren von Bakteriorhodopsin außerhalb der Lipiddoppelschicht der Membran liegen, sterisch gehindert sind und nur schwer mit den niedermolekularen Kopplungsreagenzien reagieren. Niedermolekulare Kopplungsreagenzien haben aber noch einen weiteren gravierenden Nachteil. Sie können durch den Protonenkanal bis in das aktive Zentrum von Bakteriorhodopsin vordringen. Insbesondere eine Reaktion der Linkermoleküle mit der Bindungsstelle des Retinalaldehyds an Lysin-216 (Schiff-Base) führt zu einer irreversiblen Schädigung der gewünschten Funktionen des Proteins. Deshalb sind die weit verbreiteten niedermolekularen Kopplungsreagenzien, die mit Aminogruppen reagieren, besonders nachteilig. Weiter müssen die niedermolekularen Linker im Überschuss zugegeben werden, um wenigstens eine einigermaßen akzeptable Ausbeute und Vernetzungsgeschwindigkeit zu erzielen. Eine Vielzahl nicht reagierter Moleküle bleibt im Material und müsste nach der Reaktion entfernt werden. Dies ist z.B. bei der Herstellung von optischen Filmen nicht möglich.

Die Nachteile niedermolekularer Linker sind im Folgenden kurz stichpunktartig zusammengefasst:

- Wenige Aminosäuren von Bakteriorhodopsin sind für Linker zugänglich.
- Linker, die mit funktionellen Aminogruppen reagieren, dürfen nicht eingesetzt werden.
- Nicht abreagierte Linker müssen entfernt werden, da sonst eine unkontrollierte, zeitlich lang andauernde Nachreaktion erfolgen wird, die zu einer stetigen Änderung der Materialeigenschaften und fehlender Langzeitstabilität führt.

Die EP 0 417 541 offenbart ein Verfahren zur Quervernetzung von orientierten Proteinen unter Einsatz niedermolekularer Linker wie Glutaraldehyd, Carbodiimid oder Diaminen.

B.J. Gaffney, Biochim. Biophys. Acta, 822 (1985) 289-313 ist ein Übersichtsartikel über die chemische und biochemische Quervernetzung von verschiedenen Membranbestandteilen. Der Artikel offenbart unter anderem die biochemische Quervernetzung von nicht näher spezifizierten Zytoskelett- und Membranproteinen von Erythrozyten durch zelleigene Transglutaminase bei Zugabe eines Calcium-Ionophors und von millimolarem Calcium. Eine Quervernetzung von Bacteriorhodopsin ist dagegen lediglich unter Verwendung von niedermolekularen, fotoaktivierbaren Linkern mit einer Isothiocyanatgruppe beschrieben.

Die WO 99/06446 offenbart ein Verfahren zum Transglutaminase-katalysiertem Koppeln von Proteinen oder Peptiden an einen Träger. Das Dokument gibt keinen Hinweis darauf, ob sich Bacteriorhodopsin als Substrat einer Transglutaminase eignet.

Eine Aufgabe der Erfindung bestand somit darin, ein Verfahren zur kovalenten Verknüpfung von Bakteriorhodopsin in Membranform, insbesondere zur direkten Quervernetzung von Purpurmembranen, ohne Verwendung von niedermolekularen Linkern bereitzustellen. Ein weiterer Einsatzbereich ist die Kopplung von Bakteriorhodopsin in Membranform an Oberflächen, Polymere oder Hilfsstoffe, insbesondere Farbstoffe.

Diese Aufgabe wird durch ein Verfahren zur Herstellung von kovalent vernetztem Bakteriorhodopsin gelöst, welches dadurch gekennzeichnet ist, dass Bakteriorhodopsin in membrangebundener Form als Substrat einer Transglutaminase, insbesondere einer bakteriellen Transglutaminase umgesetzt und dabei kovalent quervernetzt wird.

Unter dem Begriff Bakteriorhodopsin, wie er hierin verwendet wird, wird sowohl der Bakteriorhodopsin-Wildtyp (BR-WT) als auch Bakteriorhodopsin-Varianten verstanden. Der Begriff Bakteriorhodopsin-Varianten umfasst sowohl Bakteriorhodopsin-Moleküle, die sich von BR-WT durch Addition, Substitution, Deletion und/oder Insertion von Aminosäuren, insbesondere von mindestens einer, besonders bevorzugt von mindestens zwei, am meisten bevorzugt von mindestens drei, und bis zu 50, bevorzugt bis zu 20, und besonders bevorzugt bis zu 10 Aminosäuren unterscheiden. Weiterhin fallen unter den Begriff Varianten auch Bakteriorhodopsin-Moleküle, in denen Retinal durch retinalanaloge Moleküle ersetzt ist, sowie Bakteriorhodopsin-Moleküle, die chemisch modifiziert wurden, beispielsweise durch Einführen von Schutzgruppen oder funktionellen Seitengruppen.

Für die Zwecke dieser Erfindung umfasst der Ausdruck "Bakteriorhodopsin" auch strukturell mit Bakteriorhodopsin verwandte Membranproteine, die in membrangebundener Form vorliegen und eine Homologie von mindestens 70%, mehr bevorzugt mindestens 80%, am meisten bevorzugt mindestens 90%, zum Bakteriorhodopsin-Wildtyp aufweisen.

Homologie, wie hierin verwendet, wird definiert als

$$H\,(\%) = (1-A/[BR\text{-}WT]) \times 100,$$

worin H die Homologie in Prozent bedeutet, A die Anzahl der gegenüber der Bakteriorhodopsin-Wildtypsequenz vorhandenen Änderungen darstellt und [BR-WT] die Anzahl der Aminosäuren der Bakteriorhodopsin-Wildtypsequenz ist.

Beispiele für strukturell mit Bakteriorhodopsin verwandte Membranproteine in membrangebundener Form, die von der Erfindung umfasst sind, sind Halorhodopsin sowie Sensorrhodopsin. Diese Membranproteine liegen in der Membranform nichtkristallin vor.

Erfindungsgemäß bevorzugt einzusetzende Bakteriorhodopsin-Materialien umfassen Bakteriorhodopsin-Wildtyp sowie Bakteriorhodopsin-Varianten mit einer veränderten Aminosäuresequenz. Bevorzugt weisen die Bakteriorhodopsin-Varianten eine modifizierte physikalische Funktion auf, insbesondere eine modifizierte Lichtempfindlichkeit, eine veränderte Farbe oder eine veränderte Kinetik des photochromen Verhaltens von Bakteriorhodopsin. Am meisten bevorzugt sind Bakteriorhodopsin-Varianten, bei denen gezielt Bindungsstellen für die enzymatische Quervernetzung mit Transglutaminase deletiert bzw. eingeführt sind. Besonders bevorzugt werden Bakteriorhodopsin-Varianten verwendet, bei denen Gln oder Lys deletiert oder durch andere Aminosäuren ausgetauscht sind. In Bakteriorhodopsin befindet sich eine Lys-Bindestelle intrazellulär (= C-Terminus) an den Positionen Lys41, Lys42, Lys159 und Lys172 sowie extrazellulär (N-Terminus) an Position Lys129. Gln-Bindungsstellen finden sich in Bakteriorhodopsin extrazellulär (= N-Terminus) an den Positionen Gln3 und Gln75. Es wurde festgestellt, dass insbesondere die Aminosäuren Gln3 und Lys129 als Bindestellen bei der Umsetzung mit Transglutaminase fungieren.

Weiterhin bevorzugt sind solche Bakteriorhodopsin-Varianten, bei denen in eine Schleife Gln oder Lys so eingeführt ist, dass es für Transglutaminase zugänglich ist. Besonders bevorzugt sind solche Bakteriorhodopsin-Varianten, bei denen eine oder zwei Bindungsstellen für Transglutaminase vorliegen. Bei Vorliegen von zwei Bindungsstellen, die für die Transglutaminase zugänglich sind, können die beiden Bindungsstellen entweder auf der gleichen Membranseite oder bevorzugt auf unterschiedlichen Membranseiten angeordnet sein, d.h. eine Bindungsstelle auf der zytoplasmatischen Seite und eine auf der extrazellulären Seite. Auf diese Weise ist ein gerichtetes Übereinanderschichten von Membranen im Gegensatz zu einer statistischen Quervernetzung möglich. Durch ein gerichtetes Übereinanderschichten können Elemente erzeugt werden, in denen sich die Spannung von 300 mV pro Schicht mit der Anzahl der Schichten erhöht. Bei bisher bekannten Verfahren zur Bildung von mehrschichtigen BR-Purpurmembranen wird lediglich eine Vorzugsorientierung von etwa 10% erhalten. Erfindungsgemäß ist hingegen ein gerichtetes Übereinanderschichten mit einer deutlich höheren Vorzugsorientierung möglich. Bevorzugt werden etwa 4 bis 10 BR-Purpurmembran-Schichten übereinandergelagert, wobei eine Vorzugsorientierung um mehr als 30%, bevorzugt von mehr als 50% und besonders bevorzugt von mehr als 80% erhalten werden kann.

Weiterhin sind Bakteriorhodopsin-Varianten bevorzugt, in denen Retinal durch retinalanaloge Moleküle ersetzt ist, wodurch beispielsweise der Photozyklus des Bakteriorhodopsin-Materials verändert werden kann. Retinalanaloge Moleküle sind Moleküle, die als Chromophor im Bakteriorhodopsin eingebaut werden können und sich von Retinal z.B. durch eine Derivatisierung unterscheiden.

Eine weitere bevorzugte Klasse von Bakteriorhodopsin-Materialien sind Bakteriorhodopsin-Varianten bzw. Bakteriorhodopsin-Wildtyp, die chemisch modifiziert wurden. Geeignete Modifizierungen sind beispielsweise im US-Patent 5,922,843 beschrieben. Die Varianten können auch durch Blockierung von Aminosäuren, beispielsweise durch Schutzgruppen oder andere chemische Modifizierung der Aminosäureseitenketten modifiziert werden.

Bakteriorhodopsin-Varianten können auch durch enzymatische Vorbehandlung von Bakteriorhodopsin-Wildtyp bzw. Bakteriorhodopsin-Varianten erhalten werden. Eine enzymatische Spaltung kann beispielsweise mittels Peptidasen herbeigeführt werden, wobei insbesondere der Aminoterminus entfernt wird oder Spaltungen in den außerhalb der Membran liegenden Schleifen ("Loops") herbeigeführt werden.

Es können auch Varianten eingesetzt werden, die eine Kombination von mehreren der oben genannten Modifikationen aufweisen.

Es hat sich überraschenderweise herausgestellt, dass Bakteriorhodopsin in membrangebundener Form und insbesondere in Purpurmembran-Form ein Substrat der Transglutaminase und insbesondere der bakteriellen Transglutaminase (bTGA) ist. Dies ist insbesondere deshalb unerwartet, da, wie oben ausgeführt, die Aminosäuren des Bakteriorhodopsins in der Membranform, insbesondere in der Purpurmembran-Form, zum überwiegenden Teil innerhalb der Lipiddoppelschicht der Membran liegen und somit nicht zugänglich sind. Das Bakteriorhodopsin liegt in der Purpurmembran-Form nicht in freier Form, sondern als übergeordnete, scheibenförmige Protein-Lipid-Kristallstruktur vor. Trotzdem wird es überraschenderweise von bakterieller Transglutaminase als Substrat angenommen und umgesetzt.

Bisher war lediglich bekannt, dass lösliche Proteine, wie etwa Enzyme, an einen Träger, beispielsweise an Gelatine oder ein Markerenzym, Transglutaminase-katalysiert gekoppelt werden können. Solch ein Verfahren wird z.B. in DE 197 32 917 C1 beschrieben. Dazu werden Proteinlösungen mit Transglutaminase umgesetzt. Einen Hinweis, dass wasserunlösliche Proteine oder gar wasserunlösliche Protein-Lipid-Aggregate als Substrat von Transglutaminase angenommen werden, wird in DE 197 32 917 nicht offenbart. Noch viel weniger wird Bakteriorhodopsin oder gar Bakteriorhodopsin in Purpurmembran-Form in Erwägung gezogen.

Ein ähnliches Verfahren zur Umsetzung von wasserlöslichen Proteinen mit TGA, beispielsweise die Quervernetzung von Kasein oder Gelatine, wird auch in US 5,731,183 und US 5,948,662 beschrieben. Dazu werden Lösungen der Proteine mit den in diesen US-Patenten offenbarten speziellen Transglutaminasen umgesetzt. Eine Offenbarung oder einen Hinweis darauf, dass auch wasserunlösliche Proteine oder gar übergeordnete Strukturen als Substrat für TGA dienen könnten, findet sich nicht. Noch viel weniger wird Bakteriorhodopsin oder gar Bakteriorhodopsin in Purpurmembran-Form erwähnt.

Es wurde nun überraschenderweise festgestellt, dass man mit Hilfe von TGA Bakteriorhodopsin in der Membranform und sogar in der Purpurmembran-Form direkt und ohne Hinzunahme niedermolekularer Linkermoleküle untereinander quervernetzen kann. Weiterhin können an das Bakteriorhodopsin Polymere, auch andere Substanzen, wie etwa Oberflächen oder Hilfsstoffe, z.B. Farbstoffe, gebunden werden. Die Quervernetzung findet bei etwa 40°C statt. Durch nachfolgende Erwärmung des Reaktionsansatzes auf 80°C wird die TGA inaktiviert. Bakteriorhodopsin in Purpurmembran-Form übersteht die Wärmebehandlung unbeschadet. So kann die Reaktion gezielt gestoppt werden. Eine Entfernung von überschüssigen niedermolekularen Linkern ist nicht notwendig.

Bakteriorhodopsin-Purpurmembranen, insbesondere unterschiedliche Varianten von Bakteriorhodopsin-Purpurmembranen, können sowohl als erstes wie auch als zweites Substrat für TGA, insbesondere bTGA fungieren. Dadurch ergeben sich umfangreiche Möglichkeiten zur direkten Vernetzung von Purpurmembranen untereinander oder/und zur kovalenten Kopplung von Polymeren, Oberflächen oder/und Substraten an Bakteriorhodopsin in Purpurmembran-Form sowohl in quervernetzter als auch in nicht-vernetzter Form.

Ein Aspekt der Erfindung betrifft somit ein Verfahren zur kovalenten Verbindung von Bakteriorhodopsin in Membranform, insbesondere in Purpurmembran-Form an Polymere, Oberflächen oder/und Hilfsstoffe mittels TGA, wobei das Bakteriorhodopsin und das weitere Substrat mit einer Transglutaminase umgesetzt und miteinander kovalent verknüpft werden. Durch ein solches Verfahren können zum Beispiel kovalent verknüpfte Konjugate bestehend aus Bakteriorhodopsin in Purpurmembran-Form und einem weiteren Molekül gebildet werden. Geeignete Polymere, an die Bakteriorhodopsin in Purpurmembran-Form mittels TGA kovalent gebunden werden kann, umfassen zum Beispiel Gelatine, Polyvinylalkohol und Polyethylen, die Seitengruppen-modifiziert sein können. Geeignete Substrate oder Hilfsstoffe sind insbesondere niedermolekulare Verbindungen, wie etwa Farbstoffe.

Da erfindungsgemäß auf jegliche Linkermoleküle verzichtet werden kann, ergeben sich erhebliche wirtschaftliche und technologische Vorteile.

Neben den oben beschriebenen Bakteriorhodopsinen in membrangebundener Form kann erfindungsgemäß unter Verwendung einer Transglutaminase auch Bakteriorhodopsin in löslicher Form, insbesondere in monomerer oder trimerer Form, kovalent quervernetzt werden. Insbesondere kann Bakteriorhodopsin, wie es bei heterologer Expression von Bakteriorhodopsin gebildet wird, oder Bakteriorhodopsin, wie es durch Solubilisation von Purpurmembranen entsteht (monomerisiertes Bakteriorhodopsin), gemäß dem Verfahren der Erfindung als Substrat einer Glutaminase umgesetzt und kovalent quervernetzt werden.

Darüber hinaus ist es erfindungsgemäß auch möglich, von Bakteriorhodopsin verschiedene Membranproteine als Substrat einer Transglutaminase umzusetzen und dabei kovalent querzuvernetzen, soweit sie in membrangebundener Form vorliegen. Insbesondere können Membranproteine, die mehrere, insbesondere vier bis acht helikale (vor allem α-helikale) membrandurchspannende Aminosäureabschnitte enthalten, umgesetzt werden. Beispiele für geeignete Membranproteine sind Kanalproteine, Porter, Rezeptoren, G-Proteine usw.

Das erfindungsgemäße Verfahren kann insbesondere verwendet werden, um zwei gleiche oder unterschiedliche Bakteriorhodopsin-Materialien, wie sie oben beschrieben wurden, kovalent zu vernetzen. In einer weiteren bevorzugten Ausführungsform wird das erfindungsgemäße Verfahren eingesetzt, um ein Bakteriorhodopsin-Material kovalent an ein anderes Material zu binden. Bevorzugtes Beispiel hierfür ist die kovalente Bindung von Bakteriorhodopsin-Materialien, insbesondere Bakteriorhodopsin-Materialien in Purpurmembran-Form an eine Oberfläche, an ein Polymer oder/und an eine niedermolekulare Verbindung (hierin auch als Hilfsstoff bezeichnet).

Zur kovalenten Bindung an eine Oberfläche wird das Bakteriorhodopsin beispielsweise als Substrat der Transglutaminase umgesetzt, wobei als zweites Substrat ein Molekül verwendet wird, welches neben der Bindungsgruppe für die Transglutaminase eine weitere funktionelle Gruppe, beispielsweise eine endständige Thiol-Gruppe, aufweist. Das bei der Umsetzung mit Transglutaminase gebildete Addukt kann dann über die eingeführte funktionelle Gruppe an eine Oberfläche immobilisiert werden. Bei Einbringung einer Thiol-Gruppe kann das Bakteriorhodopsin beispielsweise kovalent an eine Goldoberfläche immobilisiert werden. Dadurch ist es möglich, eine gerichtete Immobilisierung von Bakteriorhodopsin, insbesondere in der Purpurmembran-Form, zu erreichen, da das Bakteriorhodopsin nur mit der Seite kovalent an die Oberfläche gebunden werden kann, auf der die Thiol-Gruppe eingeführt worden ist.

Daneben ist es auch möglich, Bakteriorhodopsin direkt an Oberflächen mittels Transglutaminase kovalent zu binden, soweit die Oberfläche Gruppen aufweist, die als Substrat der Transglutaminase wirken können.

Weiterhin ist es mit den erfindungsgemäßen Verfahren möglich, Bakteriorhodopsin an ein Polymer kovalent zu binden. Dabei wird ein Oligomer oder Polymer, welches natürlichen oder synthetischen Ursprungs sein kann, mit dem erfindungsgemäßen Verfahren mit Bakteriorhodopsin verknüpft. Ein Beispiel für ein natürliches Polymer ist Gelatine, ein Beispiel für ein bevorzugtes synthetisches Polymer ist Polyvinylalkohol. Voraussetzung für eine erfolgreiche Umsetzung ist, dass das Oligomer bzw. Polymer eine Seitengruppe enthält, die ein Substrat der Transglutaminase ist, also einen Glutamin(Gln)- oder einen Lysin(Lys)-Rest enthält oder ein Glutamin-Analogon oder ein Lysin-Analogon enthält oder einen Glutamin-Teilbereich oder einen Lysin-Teilbereich enthält, der von Transglutaminase als Substrat erkannt wird, insbesondere einen Teilbereich, der mindestens drei C-Atome und eine Aminogruppe umfasst.

Eine entsprechende Gruppe kann in dem Polymer bereits vorhanden sein, wie etwa in Gelatine, oder sie kann chemisch eingeführt werden.

Mit dem erfindungsgemäßen Verfahren erhält man ein Material, in dem Bakteriorhodopsin, insbesondere in der Purpurmembran-Form, an ein Polymer in stöchiometrischer Weise kovalent gekoppelt vorliegt. Bei der Verarbeitung eines Materials erhält man dadurch erhebliche Vorteile. Insbesondere kann keine Entmischung auftreten, wie sie bei lediglich physikalisch vermischten Polymeren, die an sich ineinander nicht löslich sind, häufig auftritt. Weiterhin eröffnet sich die Möglichkeit, die Verarbeitung von Bakteriorhodopsin enthaltenden Materialien in organischen Lösungsmitteln durchzuführen, wenn das Bakteriorhodopsin an ein Oligomer/Polymer gebunden wird, das sowohl in Wasser oder einem Puffer (für die enzymatische Quervernetzung) als auch in organischen Lösungsmitteln möglich ist.

In einer weiteren bevorzugten Ausführungsform wird das Bakteriorhodopsin mit einem Oligomeren/Polymeren quervernetzt, welches seinerseits noch weitere Polymerisierungsreaktionen oder Quervernetzungsreaktionen eingehen kann. Solche weiteren Quervernetzungs- bzw. Polymerisationsreaktionen können z.B. mit UV-Licht oder durch einen radikalischen Starter initiiert werden. Dadurch ergibt sich die Möglichkeit, Bulk-Materialien herzustellen, die ein homogen verteiltes Bakteriorhodopsin enthalten. Solche Materialien können vorteilhafterweise zur Herstellung von Bakteriorhodopsin-Würfeln verwendet werden, wie sie für eine dreidimensionale Datenspeicherung eingesetzt werden können. Solche Materialien werden bisher durch Zumischung von Bakteriorhodopsin bei der Polymerisation von beispielsweise auf Acrylsäure oder Acrylamid basierende Polymeren in wässrigen Lösungsmitteln hergestellt, wobei keine kovalente Quervernetzung zwischen dem Bakteriorhodopsin und dem Polymer ausgebildet wird. Bei der Entfernung des Wassers kommt es häufig zu hohem Schwund. Darüber hinaus werden bei solchen Materialien des Standes der Technik Entmischungsphänomene beobachtet, die zu einer inhomogenen Verteilung des Bakteriorhodopsins in dem Material führen. In einer weiteren bevorzugten Ausführungsform wird das Bakteriorhodopsin-Material an eine niedermolekulare Verbindung kovalent gebunden. Die niedermolekulare Verbindung ist dadurch gekennzeichnet, dass sie ein Substrat der Transglutaminase ist, also insbesondere ein Glutamin-Analogon oder ein Lysin-Analogon. Die niedermolekulare Verbindung, die hierin auch Hilfsstoff genannt wird, kann ausgewählt werden aus der Gruppe bestehend aus Farbstoffen, Fluorochromen, Lipiden, Peptiden, oligomeren und polymeren Nukleinsäuren, wie etwa DNA/RNA/PNA usw., synthetischen Oligomeren und Polymeren, Proteinen (Avidin-Biotin etc.), Lektinen, Polysacchariden, leitfähigen Polymeren und anderen. Mit dem erfindungsgemäßen Verfahren ist eine gezielte, stöchiometrische, ortsselektive Ankopplung der Verbindungen an das Bakteriorhodopsin, insbesondere an Bakteriorhodopsin in Purpurmembran-Form, möglich.

Farbstoffe und Fluorochrome werden insbesondere kovalent angekoppelt, um eine Änderung des Anfangsfarbzustandes des Bakteriorhodopsins bzw. der Purpurmembran zu erzielen. Vorteilhaft ist dabei, dass keine Entmischung zwischen Bakteriorhodopsin und Farbstoff bzw. Fluorochrom auftritt und dass die Verarbeitung nicht durch unterschiedliche Löslichkeiten der Materialien nachteilig beeinflusst wird.

Lipide können kovalent angekoppelt werden, um eine Verankerung von Bakteriorhodopsin, insbesondere in Membranform, auf einer anderen Lipidmembran zu erhalten, wobei die weitere Membran bevorzugt ebenfalls eine Purpurmembran und besonders bevorzugt ebenfalls eine Bakteriorhodopsin-Purpurmembran darstellt. Lipidgekoppelte Bakteriorhodopsin-Materialien können weiterhin vorteilhaft in der Langmuir-Blodgett-Technik eingesetzt werden.

Durch die Kopplung von Bakteriorhodopsin mit Peptiden können immunologische Tags eingebracht werden und Materialien mit selektiver, hochaffiner Bindefähigkeit (z.B. Antigen-Antikörper-Wechselwirkung) erhalten werden.

Durch die Kopplung von Bakteriorhodopsin mit Oligomeren, insbesondere oligomeren oder polymeren Nukleinsäuren, wie etwa DNA, RNA, PNA usw., ist der Aufbau dreidimensionaler gerichteter Strukturen möglich.

Durch die kovalente Kopplung von Bakteriorhodopsin mit synthetischen Oligomeren bzw. Polymeren können quervernetzte Materialien erhalten werden, die gegen Entmischung stabil sind.

Durch die kovalente Kopplung von Proteinen, wie etwa Avidin, Biotin oder ähnlichen, die insbesondere ein Partner eines selektiven, hochaffinen Bindungspaares sind, können Materialien mit hoher Komplexbildungskonstante erhalten werden.

Als leitfähige Materialien werden bevorzugt Protonen-leitfähige Materialien, wie etwa Protonen-leitfähige Polymere, oder Membranen mit dem Bakteriorhodopsin gekoppelt.

Ein weiterer Gegenstand der vorliegenden Erfindung ist ein linkerfrei kovalent vernetztes Bakteriorhodopsin, welches durch die oben beschriebenen Vorgehensweisen erhalten werden kann. Ein solches Material, welches ausschließlich aus Bakteriorhodopsin oder aus Bakteriorhodopsin und daran kovalent gebundenen weiteren Materialien bzw. Stoffen aufgebaut sein kann, weist gegenüber bekannten Bakteriorhodopsin-Materialien strukturelle Unterschiede auf, die erhebliche Vorteile liefern. Bei der erfindungsgemäßen direkten Kopplung wird kein Linker zwischengeschaltet, so dass auch in dem erhaltenen Material die Bakteriorhodopsin-Materialien direkt gebunden und nicht über Linker vernetzt sind. Dadurch können die mit der Verwendung von Linkern verbundenen Nachteile, wie sie oben beschrieben wurden, ausgeschaltet werden. Bei der Kopplung von Bakteriorhodopsin an andere Materialien wird durch die kovalenten Bindungen im Gegensatz zu im Stand der Technik bekannten physikalischen Mischungen zudem erreicht, dass stabile, homogene Werkstoffe erhalten werden können. Solche Werkstoffe sind insbesondere zur Verwendung in der Datenspeicherung geeignet. Durch die gerichtete Anordnung von mehreren Schichten von BR-Purpurmembranen mit einer hohen Vorzugsorientierung können Materialien erhalten werden, die bei photoelektrischen Anwendungen, wie sie z.B. bei F. Hong, Progress in Surface Science 62 (1999), 1-237, beschrieben werden, ein verbessertes Verhalten zeigen. Solche Materialien erzeugen höhere Spannungen bei Belichtung als bekannte, weniger gut ausgerichtete BR-Materialien und können insbesondere als Schalter oder als Steuerelemente eingesetzt werden.

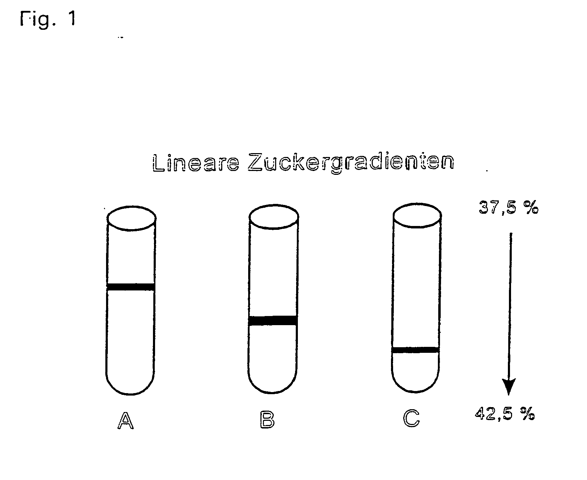

Die Erfindung wird durch die folgenden Beispiele und die Figuren weiter erläutert, wobei Fig. 1 eine schematische Darstellung der Ergebnisse einer Zuckerdichte-Gradientenzentrifugation ist. Fig. 1A zeigt Bakteriorhodopsin Wildtyp (BR-WT), Fig. 1B die Bakteriorhodopsin-Variante BRD2N und Fig. 1C miteinander quervernetztes BR-WT und BR-D2N nach 24 Stunden Inkubation mit BTGase (bakterielle Transglutaminase).

Fig. 2 zeigt eine schematische Darstellung von SDS-PAGE. A) BR-WT; B) BR-WT + BTGase, 0 Stunden inkubiert bei 37°C; C) BR-WT + BTGase, 10 Minuten inkubiert bei 37°C; D) BR-WT + BTGase, 20 Minuten inkubiert bei 37°C; E) BR-WT + BTGase, 120 Minuten inkubiert bei 37°C.

Fig. 3 zeigt die Ausbildung von Vernetzungsprodukten von BR-WT und BR-D2N in Gegenwart von bakterieller Transglutaminase in Abhängigkeit der Zeit.

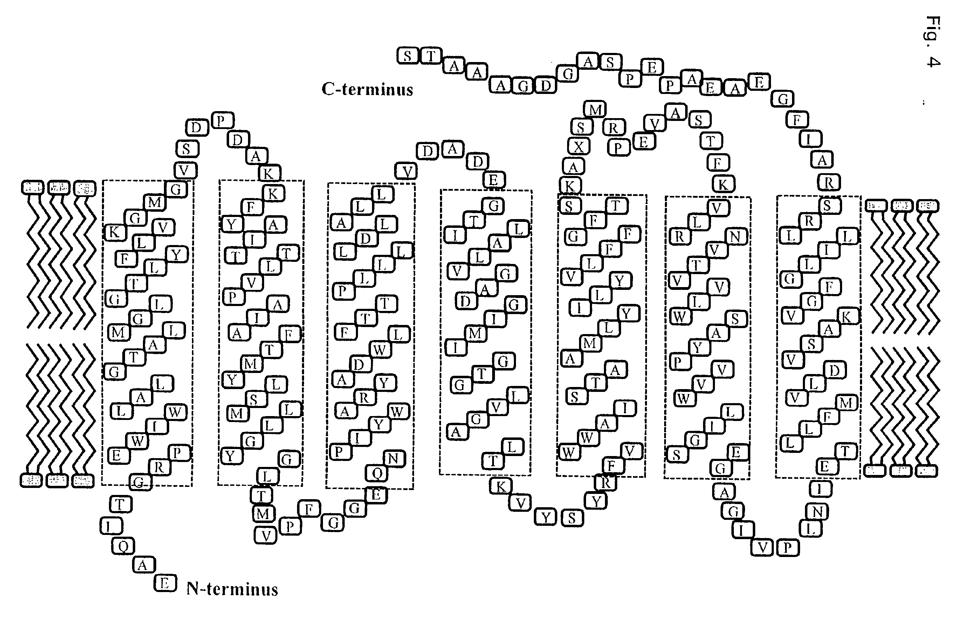

Fig. 4 zeigt die Sequenz von Bakteriorhodopsin mit der transmembranen Anordnung, modifiziert nach R.R. Birge, Photophysics and Molecular Electronic Applications of the Rhodopsins, Annu. Rev. Phys. Chem. 41 (1990), 683-733.

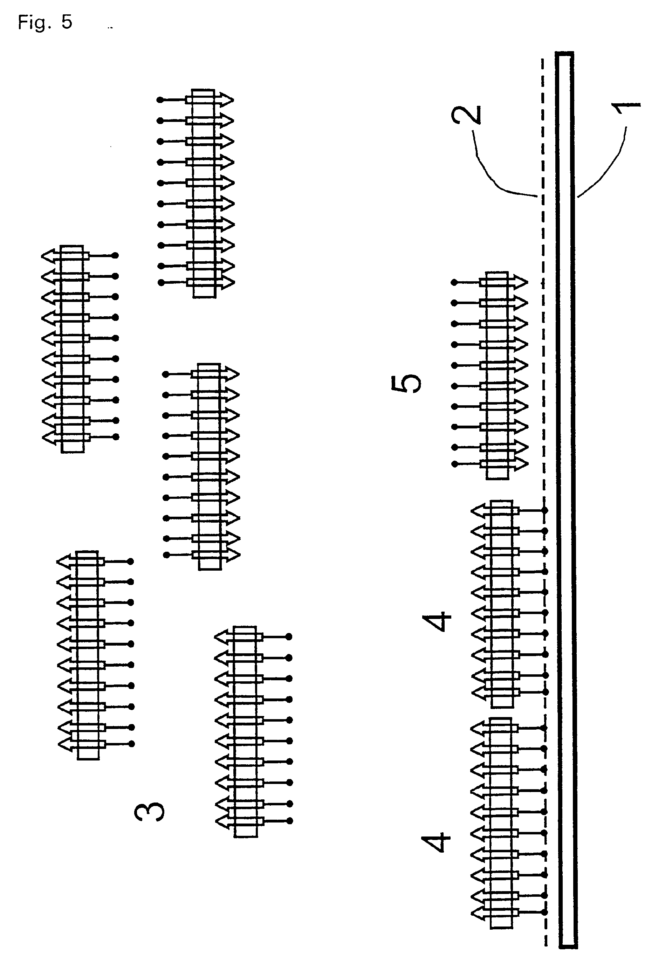

Fig. 5 zeigt ein Beispiel für eine gerichtete Abscheidung von Bakteriorhodopsin in Membranform auf Oberflächen. Auf einem Wafer 1 ist eine Gold enthaltende Schicht 2 aufgebracht. Diese Oberfläche wird mit in einem Lösungsmittel suspendierten Bakteriorhodopsin-Purpurmembranen 3 in Kontakt gebracht, die mit dem erfindungsgemäßen Verfahren Thiol-modifiziert wurden. Es findet eine Selbstorganisation 4 der Purpurmembranen in gerichteter Weise auf der Goldoberfläche statt. Purpurmembranen mit falscher Orientierung 5 werden nicht an die Oberfläche gebunden.

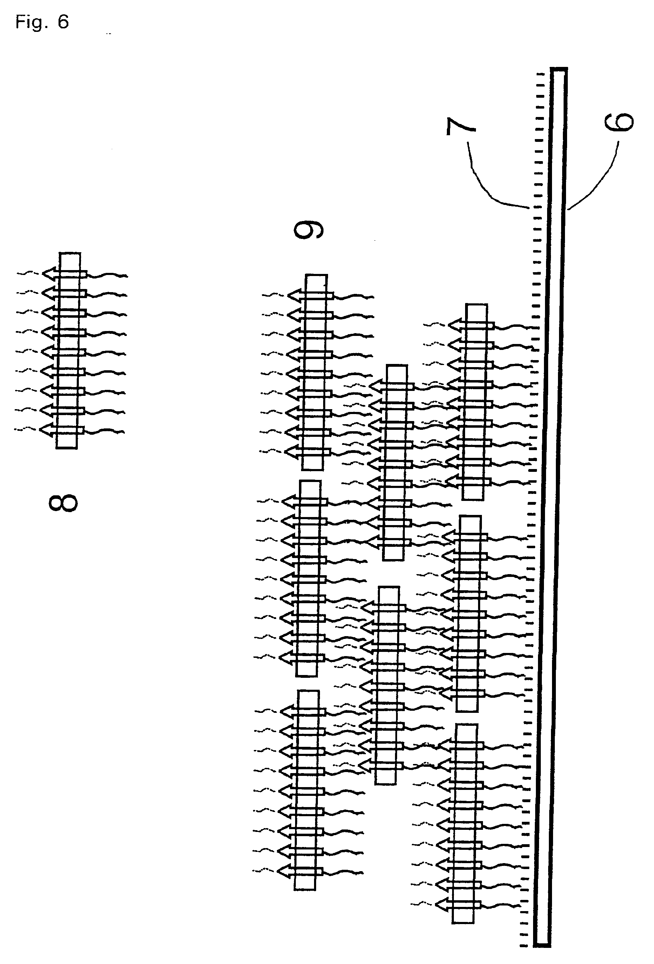

Fig. 6 zeigt ein Beispiel für eine dreidimensionale Strukturierung von Bakteriorhodopsin-Purpurmembranen. Auf eine Oberfläche oder ein Substrat 6 wird eine Haftschicht 7, z.B. Nukleinsäure-Oligomere, aufgebracht. Die Purpurmembranen 8 werden mit funktionellen Gruppen derart modifiziert, dass die auf der einen Seite der Purpurmembran-Form angebrachten funktionellen Gruppen jeweils mit den auf der anderen Seite einer weiteren Purpurmembran aufgebrachten funktionellen Gruppen wechselwirken können. Beispielsweise wird die Purpurmembran mit Nukleinsäure-Oligomeren derart modifiziert, dass die Oligomere auf beiden Seiten miteinander hybridisieren können, also die Purpurmembran extrazellulär mit der Purpurmembran-zytoplasmatisch hybridisieren kann. Auf diese Weise erfolgt eine gerichtete Bindung 9 von Purpurmembranen an Substrat und eine gerichtete Selbstorganisation der Purpurmembranen in die dritte Dimension. Auf diese Weise kann eine stark orientierte, anisotrope Schichtstruktur erhalten werden. Die von den einzelnen Purpurmembran-Formen erzeugten Photospannungen kompensieren sich jetzt nicht mehr, wie es bei einer willkürlichen Anordnung der Fall ist, sondern addieren sich. Auf diese Weise können Elemente mit einer einstellbaren Photospannung erhalten werden, welche theoretisch etwa 300 mV x Anzahl der Schichten beträgt.

Fig. 7 zeigt Purpurmembranen (gestrichelt dargestellt) mit eingebetteten Bakteriorhodopsin-Aminosäuresäulen (schematisch als durchgezogene Kästchen dargestellt). Lysin (Lys) und Glutamin (Gln), die von Transglutaminase (TGA) akzeptiert werden, sind als ausgefüllte Kreise (Lys) bzw. ausgefüllte Quadrate (Gln) eingezeichnet.

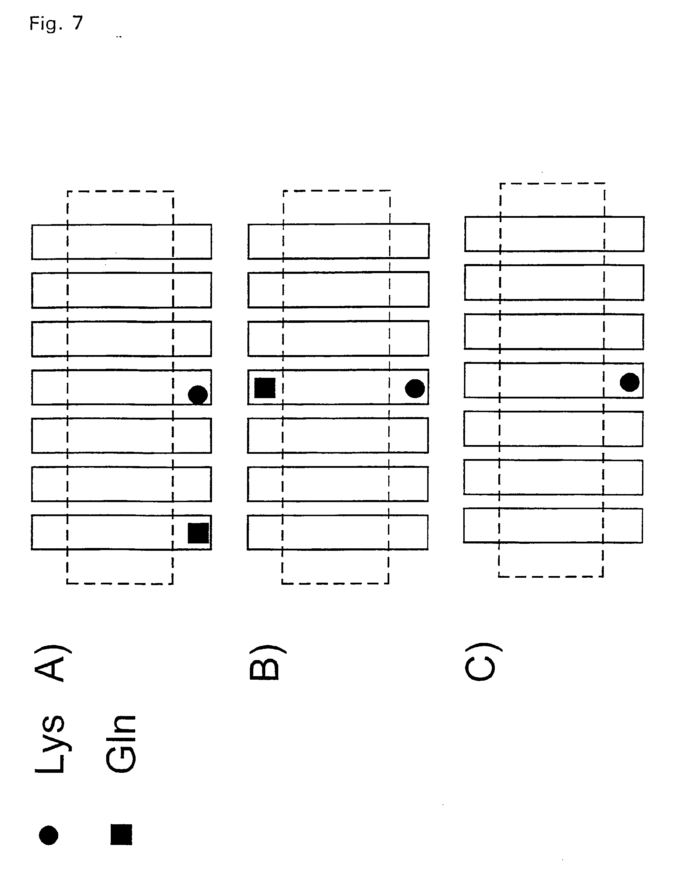

Bei Bakteriorhodopsin-Wildtyp liegen die Bindungsstellen, die für Transglutaminase zugänglich sind, nämlich Gln3 und K129, auf der gleichen Seite der Membran (Fig. 7A). In einer bevorzugten Ausführungsform wird eine Bakteriorhodopsin-Variante verwendet, die je eine Bindungsstelle auf der Innen- und eine auf der Außenseite der Membran, d.h. eine Bindungsstelle auf der zytoplasmatischen Membranseite und eine Bindungsstelle auf der extrazellulären Membranseite aufweist. Eine solche Variante wird bevorzugt dadurch erhalten, dass das Lys129 unverändert gelassen wird und das Gln3 deletiert wird und stattdessen ein Gln in einer Schleife auf der anderen Seite der Membran eingebracht wird (Fig. 7B). In einer weiteren bevorzugten Ausführungsform wird eine Bakteriorhodopsin-Variante verwendet, welche genau eine Bindungsstelle (innen oder außen) aufweist, welche beispielsweise durch Entfernen der anderen möglichen Bindungsstellen erhalten werden kann (Fig. 7C).

Beispiele:

Beispiel 1)

5 ml einer 42,5%- und 37,5%-Zuckerlösung werden verwendet, um mit einem herkömmlichen Gradientenmischer einen linearen Zuckergradienten zu erzeugen. Dieser Gradient dient zur Analyse von vernetztem und unvernetztem Bakteriorhodopsin.

Beispiel 2)

Eine Bakteriorhodopsin-Wildtype-(WT)-Lösung (20 mg/ml) und eine Lösung der Bakteriorhodopsin-Variante BR-DZN (20 mg/ml) werden mit Puffer (pH 7,0, 100 mM, Phosphatpuffer) und BTGase-(9,3 U/ml)-Lösung gemischt, so das die BTGase im Vergleich zu Bakteriorhodopsin im vierfachen Überschuss vorliegt. Die Inkubation erfolgt für 24 h bei 40°C. In der Reaktionsmischung liegt dann praktisch nur noch quervernetztes Bakteriorhodopsin vor (Abb. 1c).

Beispiel 3)

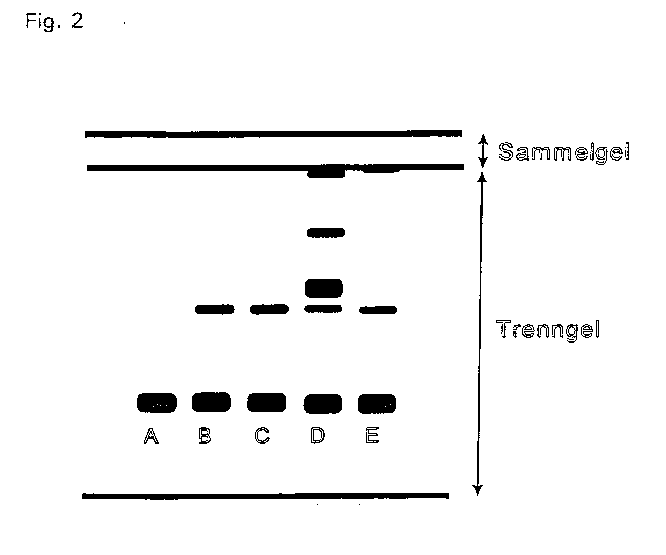

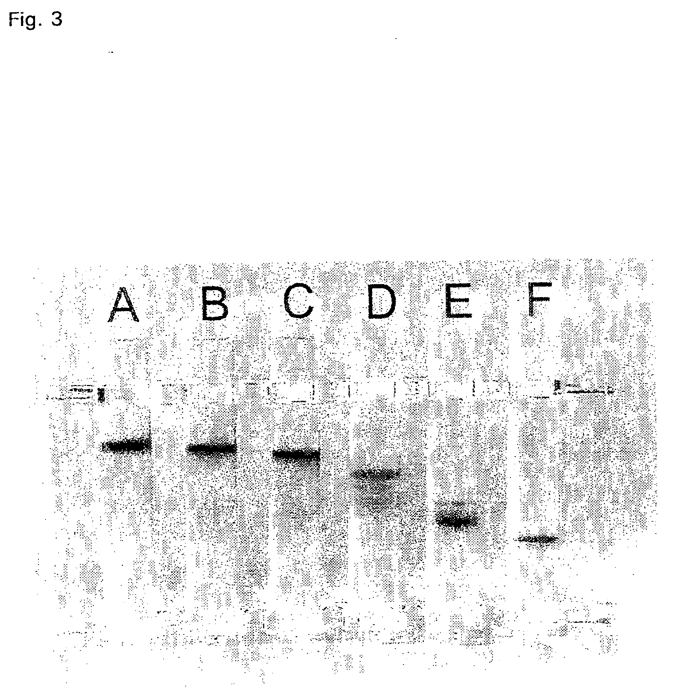

Reaktionsansatz wie bei 2). Die Reaktion wird allerdings zu einem beliebigen Zeitpunkt durch kurzfristiges Erwärmen auf 80°C gestoppt. In der Reaktionsmischung liegt dann quervernetztes und nicht quervernetztes Bakteriorhodopsin vor. Durch die Wahl des Stoppzeitpunktes können beliebige Verhältnisse zwischen vernetztem und unvernetztem Bakteriorhodopsin eingestellt werden (Abb. 2 und 3). Je höher der Vernetzungsgrad ist, desto starrer wird das quervernetzte Bakteriorhodopsin.

Die rote Bande in Abb. 3 stellt BR-WT dar, die blaue Bande BR-D2N. Durch Quervernetzung wird das lilafarbige Vernetzungsprodukt BR-WT$_x$-BR-D2N$_y$ erhalten.

Beispiel 4)

Eine Gelatinelösung (Schweineschwartengelatine), 10% (w/v) wird mit dem gleichen Volumen einer BR-Lösung (PM-Konzentration 39,1 mg/ml) und mit BTGase Lösung (12 U/ml) versetzt, so dass die Menge der BTGase mit der Menge des BR's vergleichbar ist. Die Reaktionslösung wird dann bei 37°C über Nacht inkubiert. In der Reaktionsmischung liegt das BR dann in Gelatine gebunden vor.

**Patentansprüche**

1. Verfahren zur Herstellung von kovalent vernetztem Bakteriorhodopsin,
   **dadurch gekennzeichnet,**
   **dass** Bakteriorhodopsin in membrangebundener Form als Substrat einer Transglutaminase umgesetzt und dabei kovalent quervernetzt wird.

2. Verfahren nach Anspruch 1,
   **dadurch gekennzeichnet,**
   **dass** Bakteriorhodopsin in der Purpurmembran-Form als Substrat einer Transglutaminase umgesetzt und dabei kovalent quervernetzt wird.

3. Verfahren nach Anspruch 1 oder 2,
   **dadurch gekennzeichnet,**
   **dass** es sich bei dem Bakteriorhodopsin um strukturell mit dem Bakteriorhodopsin-Wildtyp verwandte Membranproteine in membrangebundener Form, welche mindestens 90 % Homologie zum Bakteriorhodopsin-Wildtyp aufweisen, handelt.

4. Verfahren nach einem der vorhergehenden Ansprüche,
   **dadurch gekennzeichnet,**
   **dass** das Bakteriorhodopsin ausgewählt wird aus Bakteriorhodopsin-Wildtyp oder/und Bakteriorhodopsin-Varianten.

5. Verfahren nach einem der vorhergehenden Ansprüche,
   **dadurch gekennzeichnet,**
   **dass** die Bakteriorhodopsin-Varianten ausgewählt sind aus Bakteriorhodopsin-Varianten, welche sich vom Bakteriorhodopsin-Wildtyp durch Addition, Substitution, Deletion und/oder Insertion von Aminosäuren, insbesondere von mindestens einer Aminosäure, unterscheiden, Bakteriorhodopsin-Molekülen, in denen Retinal durch retinalanaloge Moleküle ersetzt ist, sowie Bakteriorhodopsin-Moleküle, die durch Einführen von Schutzgruppen oder funktionellen Seitengruppen chemisch modifiziert sind.

6. Verfahren nach einem der vorhergehenden Ansprüche,
**dadurch gekennzeichnet,**
**dass** bei den Bakteriorhodopsin-Varianten Gln oder Lys deletiert oder durch andere Aminosäuren ausgetauscht sind.

7. Verfahren nach einem der vorhergehenden Ansprüche,
**dadurch gekennzeichnet,**
**dass** gleiche oder unterschiedliche Bakteriorhodopsine oder Bakteriorhodopsin-Varianten miteinander vernetzt werden.

8. Verfahren nach einem der vorhergehenden Ansprüche,
**dadurch gekennzeichnet,**
**dass** mindestens eine Bakteriorhodopsin-Variante umgesetzt wird, bei der es sich um Halorhodopsin oder/und Sensorrhodopsin, in membrangebundener Form handelt.

9. Verfahren nach einem der vorhergehenden Ansprüche,
**dadurch gekennzeichnet,**
**dass** mindestens eine Bakteriorhodopsin-Variante umgesetzt wird, die eine gegenüber dem Bakeeterior-hodopsin-Wildtyp veränderte Aminosäuresequenz aufweist oder/und bei der Retinal durch ein Retinal-analoges Molekül ersetzt ist oder/und die chemisch oder/und enzymatische Behandlung modifiziert ist.

10. Verfahren nach Anspruch 9,
**dadurch gekennzeichnet,**
**dass** mindestens eine Bakteriorhodopsin-Variante umgesetzt wird, die nur eine einzige Bindungsstelle für Transglutaminase enthält.

11. Verfahren nach Anspruch 9,
**dadurch gekennzeichnet,**
**dass** mindestens eine Bakteriorhodopsin-Variante umgesetzt wird, die zwei Bindungsstellen für Transglutaminase enthält, die nicht auf der gleichen Membranseite liegen.

12. Verfahren nach einem der vorangehenden Ansprüche,
**dadurch gekennzeichnet,**
**dass** die Quervernetzungsreaktion durch kurzfristiges Erwärmen auf 80 °C oder darüber gestoppt wird.

13. Verfahren nach einem der vorangehenden Ansprüche,
**dadurch gekennzeichnet,**
**dass** eine bakterielle Transglutaminase verwendet wird.

14. Verfahren nach einem der vorangehenden Ansprüche,
**dadurch gekennzeichnet,**
**dass** eine bakterielle Transglutaminase verwendet wird, die ohne Cofaktor aktiv ist.

15. Verfahren nach einem der vorangehenden Ansprüche,
**dadurch gekennzeichnet,**
**dass** das Bakteriorhodopsin mit einem Polymeren, einer Oberfläche oder/und einem Hilfsstoff quervernetzt wird, **dadurch gekennzeichnet, dass** der Hilfsstoff ausgewählt wird aus der Gruppe bestehend aus Farbstoffen, Fluorochromen, Lipiden, Peptiden, Nukleinsäuren, synthetischen Oligomeren und Polymeren, Proteinen, Lektinen, Polysacchariden und leitfähigen Molekülen.

16. Linkerfrei kovalent vernetztes Bakteriorhodopsin, erhältlich nach einem der vorhergehenden Ansprüche.

17. Verwendung des linkerfrei kovalent vernetzten Bakteriorhodopsins nach Anspruch 16 für photoelektrische Anwendungen.

18. Verwendung des linkerfrei kovalent vernetzten Bakteriorhodopsins nach Anspruch 16 zur dreidimensionalen Datenspeicherung.

**Claims**

1. A process for the production of covalently cross-linked bacteriorhodopsin,
**characterised in that**
bacteriorhodopsin is reacted in a membrane-bound form as a substrate of a transglutaminase and is covalently cross-linked thereby.

2. The process according to claim 1,
**characterised in that**
bacteriorhodopsin is reacted in the purple membrane form as a substrate of a transglutaminase and is covalently cross-linked thereby.

3. The process according to claim 1 or 2,
**characterised in that**
the bacteriorhodopsin is a membrane protein in a membrane-bound form that is structurally related to wild-type bacteriorhodopsin and has at least 90% homology to wild-type bacteriorhodopsin.

4. The process according to any one of the preceding claims,
**characterised in that**
the bacteriorhodopsin is selected from wild-type bacteriorhodopsin or/and bacteriorhodopsin variants.

**5.** The process according to any one of the preceding claims,
**characterised in that**
the bacteriorhodopsin variants are selected from: bacteriorhodopsin variants that differ from wild-type bacteriorhodopsin by addition, substitution, deletion and/or insertion of amino acids, in particular of at least one amino acid; bacteriorhodopsin molecules in which retinal is replaced by retinal-like molecules; and bacteriorhodopsin molecules that are chemically modified by introduction of protecting groups and/or functional side groups.

**6.** The process according to any one of the preceding claims,
**characterised in that**
Gln or Lys are deleted or substituted by other amino acids in the bacteriorhodopsin variants.

**7.** The process according to any one of the preceding claims,
**characterised in that**
identical or different bacteriorhodopsins or bacteriorhodopsin variants are cross-linked with one another.

**8.** The process according to any one of the preceding claims,
**characterised in that**
at least one bacteriorhodopsin variant is reacted which is halorhodopsin or/and sensorrhodopsin in a membrane-bound form.

**9.** The process according to any one of the preceding claims,
**characterised in that**
at least one bacteriorhodopsin variant is reacted which has a modified amino acid sequence compared to the wild-type bacteriorhodopsin or/and in which retinal is replaced by a retinal-like molecule or/and which is chemically modified or/and which is modified by enzymatic treatment.

**10.** The process according to claim 9,
**characterised in that**
at least one bacteriorhodopsin variant is reacted which contains only a single binding site for transglutaminase.

**11.** The process according to claim 9,
**characterised in that**
at least one bacteriorhodopsin variant is reacted which contains two binding sites for transglutaminase which are not on the same side of the membrane.

**12.** The process according to any one of the preceding claims,

**characterised in that**
the cross-linking reaction is stopped by briefly heating to 80 °C or above.

**13.** The process according to any one of the preceding claims,
**characterised in that**
a bacterial transglutaminase is used.

**14.** The process according to any one of the preceding claims,
**characterised in that**
a bacterial transglutaminase is used which is active without a cofactor.

**15.** The process according to any one of the preceding claims,
**characterised in that**
the bacteriorhodopsin is cross-linked with a polymer, a surface or/and an auxiliary substance, **characterised in that** the auxiliary substance is selected from the group consisting of dyes, fluorochromes, lipids, peptides, nucleic acids, synthetic oligomers and polymers, proteins, lectins, polysaccharides and conductive molecules.

**16.** Linker-free covalently cross-linked bacteriorhodopsin obtainable by the process according to any one of the preceding claims.

**17.** Use of the linker-free covalently cross-linked bacteriorhodopsin according to claim 16 for photoelectric applications.

**18.** Use of the linker-free covalently cross-linked bacteriorhodopsin according to claim 16 for three-dimensional data storage.

**Revendications**

**1.** Procédé de préparation de bactériorhodopsine à réticulation covalente,
**caractérisé en ce que**
l'on fait réagir la bactériorhodopsine, dans sa forme intégrée à la membrane, de manière à ce qu'elle constitue le substrat d'une transglutaminase et subisse une réticulation covalente.

**2.** Procédé selon la revendication 1,
**caractérisé en ce que**
l'on fait réagir la bactériorhodopsine, sous forme de membrane pourpre, de manière à ce qu'elle constitue le substrat d'une transglutaminase et subisse une réticulation covalente.

**3.** Procédé selon les revendications 1 ou 2,
**caractérisé en ce que**

la bactériorhodopsine correspond à des protéines membranaires, dans leur forme intégrée à la membrane, dont la structure est proche du type naturel de la bactériorhodopsine, leur homologie avec le type naturel de la bactériorhodopsine étant d'au moins 90 %.

4. Procédé selon l'une des revendications précédentes,
   **caractérisé en ce que**
   la bactériorhodopsine est choisie parmi le type naturel bactériorhodopsine et/ou des variantes de la bactériorhodopsine.

5. Procédé selon l'une des revendications précédentes,
   **caractérisé en ce que**
   les variantes de bactériorhodopsine étant choisies parmi les variantes de bactériorhodopsine qui se distinguent du type naturel de la bactériorhodopsine par l'addition, la substitution, la délétion et/ou l'insertion d'acides aminés, notamment d'au moins un acide aminé, les molécules de bactériorhodopsine dans lesquelles le rétinal a été remplacé par des molécules analogues au rétinal, ainsi que les molécules de bactériorhodopsine ayant subi une modification chimique qui consiste en l'introduction de groupes protecteurs ou de groupes fonctionnels latéraux.

6. Procédé selon l'une des revendications précédentes,
   **caractérisé en ce que**,
   chez les variantes de bactériorhodopsine, Gln ou Lys sont délétées ou remplacées par d'autres acides aminés.

7. Procédé selon l'une des revendications précédentes,
   **caractérisé en ce que**
   l'on soumet à la réticulation des bactériorhodopsines ou variantes de bactériorhodopsine qui sont identiques ou différentes.

8. Procédé selon l'une des revendications précédentes,
   **caractérisé en ce que**
   l'on fait réagir au moins une variante de bactériorhodopsine s'agissant de l'halorhodopsine et/ou de la sensorrhodopsine dans leur forme intégrée à la membrane.

9. Procédé selon l'une des revendications précédentes,
   **caractérisé en ce que**
   l'on fait réagir au moins une variante de bactériorhodopsine qui présente, par rapport au type naturel de la bactériorhodopsine, une séquence d'acides aminés modifiée et/ou chez laquelle le rétinal a été remplacé par une molécule analogue au rétinal et/ou qui a subi une modification par un traitement chimique et/ou enzymatique.

10. Procédé selon la revendication 9,
    **caractérisé en ce que**
    l'on fait réagir au moins une variante de bactériorhodopsine qui ne contient qu'un seul site de liaison pour une transglutaminase.

11. Procédé selon la revendication 9,
    **caractérisé en ce que**
    l'on fait réagir au moins une variante de bactériorhodopsine qui contient deux sites de liaison pour une transglutaminase lesquels ne sont pas situés sur le même côté de la membrane.

12. Procédé selon l'une des revendications précédentes,
    **caractérisé en ce que**
    la réaction de réticulation est arrêtée par un réchauffement de courte durée à 80 °C ou plus.

13. Procédé selon l'une des revendications précédentes,
    **caractérisé en ce que**
    l'on utilise une transglutaminase bactérienne.

14. Procédé selon l'une des revendications précédentes,
    **caractérisé en ce que**
    l'on utilise une transglutaminase bactérienne qui est active sans cofacteur.

15. Procédé selon l'une des revendications précédentes,
    **caractérisé en ce que**
    la bactériorhodopsine est réticulée avec un polymère, une surface ou/et un agent auxiliaire, **caractérisé en ce que** ledit agent auxiliaire est choisi dans le groupe constitué par les colorants, les fluorochromes, les lipides, les peptides, les acides nucléiques, les oligomères et polymères synthétiques, les protéines, lectines, les polysaccharides et les molécules conductrices.

16. Bactériorhodopsine à réticulation covalente sans groupe de liaison, pouvant être obtenue selon l'une des revendications précédentes.

17. Utilisation de la bactériorhodopsine à réticulation covalente sans groupe de liaison selon la revendication 16, pour des applications photoélectriques.

18. Utilisation de la bactériorhodopsine à réticulation covalente sans groupe de liaison selon la revendication 16, pour le stockage de données tridimensionnel.

Fig. 1

Lineare Zuckergradienten

37,5 %

A    B    C    42,5 %

Fig. 2

Sammelgel

Trenngel

A    B    C    D    E

Fig. 3

Fig. 4

Fig. 5

Fig. 6

Fig. 7

Lys ●
Gln ■

A)
B)
C)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0417541 A
- WO 9906446 A
- US 5922843 A
- DE 19732917 C1
- DE 19732917
- US 5731183 A
- US 5948662 A

### In der Beschreibung aufgeführte Nicht-Patentliteratur

- **B.J. GAFFNEY.** *Biochim. Biophys. Acta,* 1985, vol. 822, 289-313
- **F. HONG.** *Progress in Surface Science,* 1999, vol. 62, 1-237
- **R.R. BIRGE.** Photophysics and Molecular Electronic Applications of the Rhodopsins. *Annu. Rev. Phys. Chem.,* 1990, vol. 41, 683-733